Europäisches Patentamt

European Patent Office

Office européen des brevets

(19)

(11) Publication number : **0 479 582 A1**

(12) **EUROPEAN PATENT APPLICATION**

(21) Application number : **91309055.1**

(22) Date of filing : **02.10.91**

(51) Int. Cl.⁵ : **A61K 6/00, A61K 9/127**

(30) Priority : **03.10.90 US 592414**

(43) Date of publication of application :
**08.04.92 Bulletin 92/15**

(84) Designated Contracting States :
**AT BE CH DE DK ES FR GB GR IT LI LU NL SE**

(71) Applicant : **Overweg, Alexander Karl Friedrich**
**Wilhelm**
**2622 Baycrest**
**Nassua Bay, Texas (US)**

(71) Applicant : **Vogel, James John**
**3507 Bayshore Blvd.**
**Baycliff, Texas 77518 (US)**

(72) Inventor : **Streckfuss, Joseph Larry**
**3407 Blue Candle Spring**
**Texas 77388 (US)**
Inventor : **Wheatcroft, Merrill Gordon**
**3507 Bayshore Blvd.**
**Baycliff, Texas 77518 (US)**
Inventor : **Overweg, Alexander Karl Friedrich**
**Wilhelm**
**2622 Baycrest**
**Nassua Bay, Texas (US)**
Inventor : **Vogel, James John**
**5542 Valkeith**
**Houston, Texas 77096 (US)**

(74) Representative : **Atkinson, Peter Birch et al**
**Marks & Clerk Suite 301 Sunlight House Quay**
**Street**
**Manchester M3 3JY (GB)**

(54) Restorative periodontal technique.

㊗ In a preferred embodiment, liposomes having multiple layers of phospholipid encapsulating antibiotic carrying aqueous layers are mixed with hydroxyapatite and collagen are placed into the area of resorbed jawbone to generate new bone tissue.

EP 0 479 582 A1

This invention pertains to the field of the treatment of infections which initiate bone resorption, more particularly to the treatment of surface tissue infections and subsequent bone resorption and more particularly to the treatment of periodontal infection by restoring the resorped boned associated therewith.

The mammalian mouth, and in particular the human mouth, harbors countless bacterial agents. Bacteria require moisture and nutrients in order to grow and reproduce. Therefore, in places in the mouth where optimum growth conditions are present, bacteria tend to multiply, at time asymptomatically. One such location in the mouth is at the gum line where the gum meets the base of the tooth below the crown. Soluble nutrients build up on the teeth adjacent the gum line and serves as a growth medium for bacteria. The mouth harbors various bacteria which are highly developed to live and multiply at the gum tooth interface. In particular, certain oral spirochetes, rods, cocci, streptococci and other motile and non-motile forms congregate in the mouth at the edge of the gum where it meets the tooth and in the space immediately below the interface of the gum and tooth. Under certain circumstances, particularly where plaque accumulates at the interface of the tooth where the tooth enters the gum, the various bacteria in the mouth can begin growing out of control. Here is a synergistic dependence among the various bacteria. For example, one type of oral cavity bacteria effluxes vitamin K as a waste product which a second type requires as a nutrient. As the various bacteria grow and multiply at equilibrium, their waste products can attack the interface of the tooth and gum down to the jawbone. The early stages of this process is commonly known as gingivitis, which leads to periodontal disease.

Periodontal disease is a debilitating dental disease process for which there is no known cure. As the gingivitis progresses, a pocket forms in the gum adjacent the tooth where the gum has eroded adjacent the tooth. The pocket is created where the localized gum pulls away from the tooth, which reaction occurs as a result of the irritation caused by the infectious agents. The bacteria continue to multiply and produce waste byproducts, and the pocket deepens, until the pocket eventually destroys the supporting structures of the teeth, destabilizing them. The bacterial waste products attack the bone structure, causing bone loss, or resorption. If left untreated, permanent tooth loss will result from periodontal disease.

The onset of periodontal disease is commonly approached with mechanical techniques and/or chemotherapeutic treatment. Mechanical techniques comprise scaling and root planing and may require cutting of the gum to expose the area of accretions below the gum line, or surgical removal of diseased tissue. Mechanical treatment is commonly combined with chemotherapeutic treatment, where the intent of the chemotherapy is to attempt to delay or eliminate the onset of reinfection so as to help the tissue adjacent the tooth to heal. One technique includes the placement of crushed bone or other inert material in the eroded area of bone, in hope that the gum will grow thereover and reattach to the tooth. It should be appreciated that reinitiation of the infection prior to the possible reattachment of the gum retards or stops the healing process.

Dentists have resorted to two types of non-mechanical chemotherapeutic periodontal disease treatment: topical applications of anti-bacterial agents, such as antibiotics, or the use of systemic antibiotics. In either case, the treatment merely arrests the advancement of the disease process and is not a cure.

The goal of antibiotic treatment can be two-fold. The antibiotic can be directed to destroy all bacteria in the infected site or simply to destroy specific bacteria leaving the remaining bacteria viable. As the deleterious effects of the infection are a result of the combination of bacteria, selective elimination of one of the symbiotic bacteria partners in the infection puts the disease process in remission. The disease process will reinitiate when each of the different bacteria species necessary for the symiosis infection return to the pocket. As each of the bacteria required can be found in other parts of the mouth or gum, it is only a matter of time before they recongregate and reinitiate their infection. However, if the severity of the repeated infections can be reduced, or if the site can be rehabilitated before the infection returns, the deleterious affects of the infection can be eliminated or controlled. Since the rehabilitation techniques, including attempting to reinstate tooth support with powdered bone and growth of attendant gum surfaces to reattach to the tooth and to eliminate the pocket can take weeks, there is a need for a safe, effective method of preventing quick reinfection which does not interfere with attempts to alter the physical geography of the mouth adjacent the infection.

Treatment of periodontal disease by systemic antibiotic treatment requires the patient to enter into a regimen of taking an antibiotic orally, i.e., through his digestive system, or intravenously or interparenterally where it will be absorbed into the bloodstream. The drug will be carried throughout the body, and some portion thereof will be transmitted to the capillaries adjacent the site of periodontal infection where it will hopefully kill the bacteria into which it comes in contact. As the antibiotic is transmitted throughout the body, it should be appreciated that far more antibiotic is ingested than reaches the periodontal infection site. Therefore, the rest of the body is subjected to the presence of the antibiotic. This can lead to side effects, such as the death and elimination of beneficial bacteria, or flora, in the digestive system. In pregnant women, systemic treatment of certain antibiotics are known to stain the fetus' teeth in utero. Also, some antibiotics, in dosages required to treat infections systemically, cause birth control pills to lose their effectiveness. Further, the antibiotic kills only a portion of the present bacteria. New bacteria continuously enter the pocket, and in combination with the remaining surviving bacteria, re-initiate the infection. As a result, the treatment must be continuously repeated over the person's lifetime. It is not uncommon for periodontal disease patients to take a course of an antibiotic, such as Tetracycline, twice a year.

Topical treatment to arrest the periodontal disease takes several forms. The most simple treatment is the use of a mouthwash containing Chlorhexidine, which is a bactericide. This material may also be directly irrigated to the infected area of the gum. When the chlorhexidine comes into contact with tooth pellicle, which is a denatured component of saliva, it turns the pellicle black. Finally, the Chlorhexidine which actually gets into the pocket will typically leave the pocket within a short time, thereby leaving the infection site unprotected against reinitiation of the infection.

A second known topical treatment includes the use of an antibiotic, such as Tetracycline, irrigated onto the infected site, or soaked into gauze or other packing which is then placed in the infected area. To place the packing, the gum may need to be cut, the packing placed on the active infection against the bone, and the gum sutured shut over the packing. The packing must be removed by reversing these steps. Another technique, using an antibiotic impregnated thread which is wrapped around the tooth and into the pocket, is also known. Topical antibiotic treatments have two basic limitations. Topically irrigated antibiotic normally least, at the site of infection, less than 24 hours unless a carrier is used. This is insufficient time to permit the site to heal before the onset of reinfection, thereby preventing or limiting the use of reconstructive processes. Second, where a carrier such as an antibiotic soaked packing is used, the packing must be both surgically placed and removed. This interferes with any proposed reconstruction of the gum to eliminate the pocket.

One overriding problem with both systemic and irrigated treatment with many antibiotics is the toxicity of many potent antibiotics. This toxicity restricts the dosage, and therefore the efficacy of these drugs when used systemically or irrigated topically.

Once the disease process has progressed to the stage where substantial bone loss has occurred, the teeth may become mobile, or loose, within their sockets in the jawbone. The teeth are retained in the jawbone by collagen fibers which extend from the root of the tooth through the periodontal membrane and into the jawbone. These fibers help retain the tooth in place, and also prevent rotation of the tooth in the jawbone socket. Extension of an untreated infection along the tooth root destroys these collagen fibers and the periodontal membrane and eventually the tooth will loosen and need to be removed.

Historically, the treatment for advanced periodontal disease was limited to removing the affected teeth. Techniques developed over the past decade have obviated the need For removal of the affected teeth in all but the most extreme cases. However, these techniques have inherent limitation which affect the effectiveness.

Hydroxyapatite, a calcium phosphate material, is used to stimulate bone regeneration in resorbed jawbone adjacent periodontal infection sites to rebuild the substructure adjacent the tooth to resupport the tooth. The use of hydroxyapatite is but one step in the overall process of restoring the bone defect by eliminating the pocket. It is believed that the body mistakes the hydroxyapatite for bone, and spontaneously regenerates new bone in place of this "old" bone material. The body is constantly in the process of replacing, and rebuilding the bone, and the use of hydroxyapatite apparently mimics the qualities of bone in need of rebuilding. However, placement of the hydroxyapatite, and retention of the material at the resorption site, are substantially limited by the structure of the material. The hydroxyapatite is a granular, powdered material, which is wetted and packed into the bone defect. It tends to migrate from the site at which it is placed. This migration prevents bone augmentation in the resorbed area. Further, the use of the hydroxyapatite requires sculpting, or forming, by the dental surgeon, so that the bone regeneration conforms to the bone profile prior to resorption. The wetted hydroxyapatite is difficult pack, as the adhesion of adjacent particles is relatively low, and as the dental tool is pressed against the hydroxyapatite adjacent the bone defect, the wetted mixture simply washes to the side of the bottom of the tool and out of the defect. This difficulty in handling makes the wetted Hydroxyapatite difficult to work with. If the hydroxyapatite migrates, the resulting regenerative form of the regenerated bone will not conform to the original profile. This creates the need for a secondary treatment of hydroxyapatite, which again may migrate, or removal of the misformed portions of the regenerated bone.

Placement of the hydroxyapatite typically requires cutting of a flap in the gum adjacent the packed, to expose the resorbed portion of the jawbone to the dental surgeon. The hydroxyapatite is mixed with a saline solution or blood, and is then packed into the defect and the flap is sutured back in place. It should be appreciated that this cutting of the gum adjacent an infected area, can cause the infection to spread to the recently disturbed area. As a precaution, some authorities recommend a prophylactic course of antibiotic while undergoing restorative surgery. This may include localized antibiotic washes or systemic antibiotic treatment, with their attendant limitations. If infection continues or recurs at the treatment site, the seriousexsudate response of the body will tend to dislodge the hydroxyapatite at the resorbed area, destroying the treatment.

One attempt to overcome the migration problem inherent in hydroxyapatite therapy involves the use of collagen as a support matrix to help maintain the hydroxyapatite granules in close proximity during the regenerative process. It has been claimed that the collagen is also resorbed and replaced with new collagen support fibers which are lost during the course of the periodontal infection. The new collagen also serves to support the tooth in place after being reformed. Further, if infection continues or recurs, the mixture will tend to be rejected from the body.

Liposomes are multilamellar bodies which have been used to deliver medical agents systematically within the human body. For example, recent publications indicate that oxygen enriched liposomes may be a practicable alternative for blood plasma for emergency situations. Insight, Dec. 1989 - Jan. 1990, pp. 44-46. Likewise, it is known that liposomes may be engineered such that once in the bloodstream, they will congregate at specific sites within the body. For example, it has been proposed that liposomes carrying medicinal agents could be structured such that the bloodstream would direct them to the lungs, liver, etc. avoiding other parts of the body. Once at the intended site, macrophages within the human body attempt to consume them, releasing whatever medicine they carry. This permits the use of medications which are otherwise harmful in the human body and/or severely reduces the amount of medication necessary to affect the desired result. Such designer liposomes may be given intravenously or taken orally, where they are absorbed into the bloodstream. U.S. Patent No. 4,762,720, Jizomoto, Aug. 9, 1988, discloses several methods of making liposomes having various medicinal agents therein. That patent also discusses oral ingestion or parenteral administration of these liposomes. Liposomes have been also used in cosmetics to hold the cosmetic agents such as moisturizers on the skin for an extended period of time. Although the use of liposomes to carry chemotherapeutic agents to target areas in the body such as the heart, cancer sites, etc., is well known. It is not known how to direct such liposomes through the bloodstream to a peripheral infection site such as the mouth. Therefore, the oral ingestion or parenteral administration of liposomes is not advantageous over routine topical or systemic chemotherapy in treating parenteral infection sites.

SUMMARY OF THE INVENTION

The present invention is a tine release topical drug administration and bone regeneration system employing multilamellar liposomes having multiple encapsulating layers of aqueous dissolved antibiotic mixed in suspension with hydroxyapatite and collagen.

When used to treat periodontal disease, liposomes according to the present invention are placed into the resorbed area of the bone created by the periodontal infection. The mixture is sculpted in place after the gum is cut to expose the resorbed bone area. The liposomes bind to hydroxypatite and collagen. It has been observed that the liposomes help hold the hydroxyapatite and collagen in place, even in the presence of minor bleeding or serousexudation. The gum is sutured, and the liposomes release a chemotherapeutic agent over a period of several days to reduce the likelihood of reinfection.

The liposome/collagen/hydroxyapatite mixture may be used in conjunction with liposomes encapsulating a chemotherapeutic agent placed into the periodontal pocket adjacent the resorbed area. This will help reduce the likelihood of reinitiation of the active infection.

In addition to generating a three fold increase in the duration of the antibiotic on site as compared to simple irrigation with antibiotics, the liposome carrier permits the use of a smaller overall volume of dosage, and at the same time a greater overall local dosage, of the selected antibiotic. The topical application of free antibiotic, in a rinse or other carrier, results in tittle antibiotic reaching the infection site. The majority of the antibiotic is swallowed or absorbed by the mouth aspirated or expectorated. With the use of liposomes, nearly all of the antibiotic is deposited at the site of infection. If free antibiotic is irrigated into the pocket, most of the antibiotic will leech or effuse from the pocket or degrade within 48 hours. Certain antibiotics, which when used systemically in high dosages are toxic or have detrimental side effects, can be used with the liposome carrier when deposited locally or topically without toxic effects. Thus, it is possible to provide localized high concentrations of antibiotics not usually attained by topical, oral, intravenous, or intramuscular routes to dental target areas which will produce immediate desired effects. Further, when combined with hydroxyapatite and collagen, the liposome will maintain antibiotic treatment at the infection site while the body acclimates the hydroxyapatite and forms new bone, without the need to periodically retreat the area with antibiotics or place the patient on a regimen of oral antibiotics.

These and other objects and advantages of the invention will become apparent upon review of the accompanying brief description of the preferred embodiment, when read in conjunction with the accompanying drawings, wherein:

DESCRIPTION OF THE DRAWINGS

Figure 1 is a partial cutaway view of the mouth showing the tooth, gum and bone prior to the onset of periodontal disease;

Figure 2 is a partial view of the mouth of Figure 1, showing periodontal disease along the tooth gum interface;

Figure 3 is a cross-sectional view of a liposome;

Figure 4 is a partial cutaway view of the mouth of Figure 2, showing the liposome hydroxyapatite paste on the jawbone;

Figure 5 is a partial cutaway view of the mouth of Figure 2 showing the reconstituted bone after treatment with the hydroxyapatite liposome collagen paste; and,

Figure 6 is a partial side view of the mouth of Figure 1 showing the flap cut into the gum to reach the bone defect.

## DESCRIPTION OF THE PREFERRED EMBODIMENT

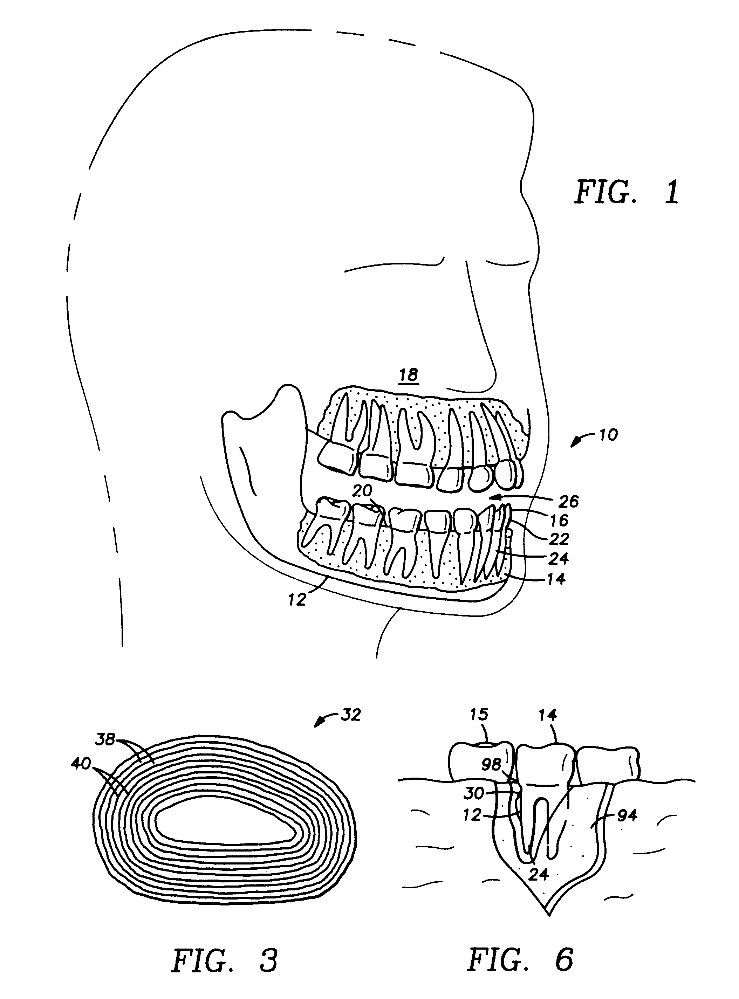

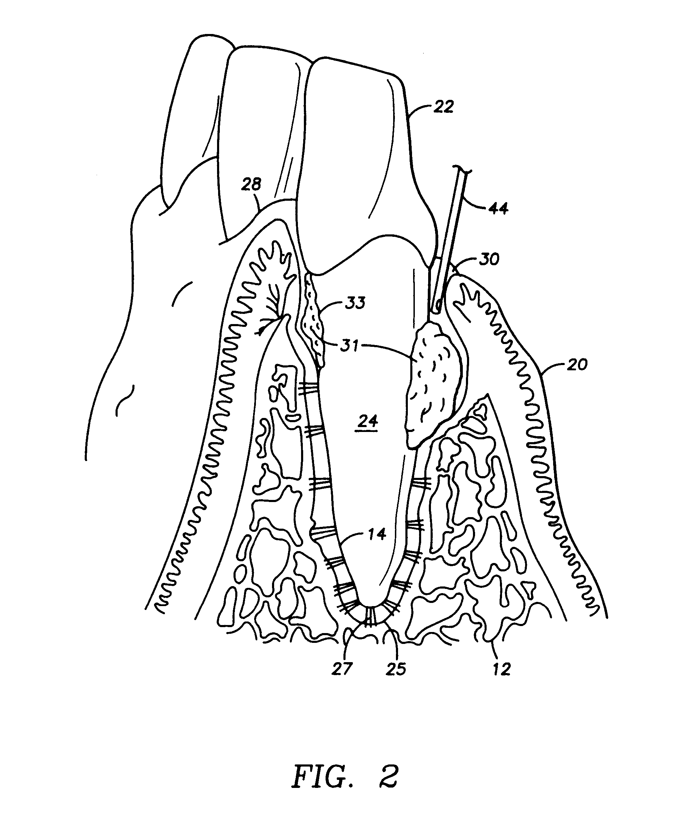

Referring to Figures 1 and 2, mouth 10 includes a jawbone 12 supporting teeth 16 therein. Teeth 16 are also supported below maxillary sinus 18 in a periodontal membrane 14 thereon. Gum 20 covers and protects jawbone 12, and extends between adjacent teeth 16.

Each tooth 16 includes an enamel crown 22 extending into mouth cavity 26 through gum 20, supported over a root 24 which extends through gum 20 to anchor tooth 16 in periodontal membrane 14. To protect the jawbone 12 from the mouth environment, periodontal membrane 14 coats the area of the gum adjacent the tooth and extends along the tooth the entire surface of the root 24 extending into gum 20. A periosteal membrane 25 coats the surface of jawbone 12, and is in surrounding contact with periodontal membrane 14. Collagen fibers 27 project from root 24 through periodontal membrane 14 into jawbone to anchor tooth 16 in place.

During the course of time, bacterial growth at interface 28 of gum 20 at root 214 leads to inflammation, which if left unchecked, leads to a swelling of gum 20 at interface 28. As the bacterial growth continues and leads to infection, the gum 20 at interface 28 pulls away from tooth 16, forming pocket 30. Pus and bacteria 31 collect in pocket 30, further irritating the gum. 20 which in turn increases the size of pocket 30. The waste products of the bacteria build up on the surface of root 24, forming scale 33 which further irritates gum 20. Pocket 30 forms as the bacterial infection attacks the periodontal membrane 14, and jawbone 12, weakening the anchoring of tooth 16 in mouth 10. Pocket 30 forms, in part, because the waste products of the bacteria leech away bone from jawbone 12. The periodontal membrane 14 is destroyed at the local site of the infection, leaving jawbone 12 reabsorbed and decalcified adjacent pocket 30. If the infection goes on unchecked, pocket 30 can extend to the base of root 24, destroying the local jawbone 12 and supporting collagen fibers 27 and membranes 14, 25 thereby weakening tooth 14 in gum 20 leading to tooth loss.

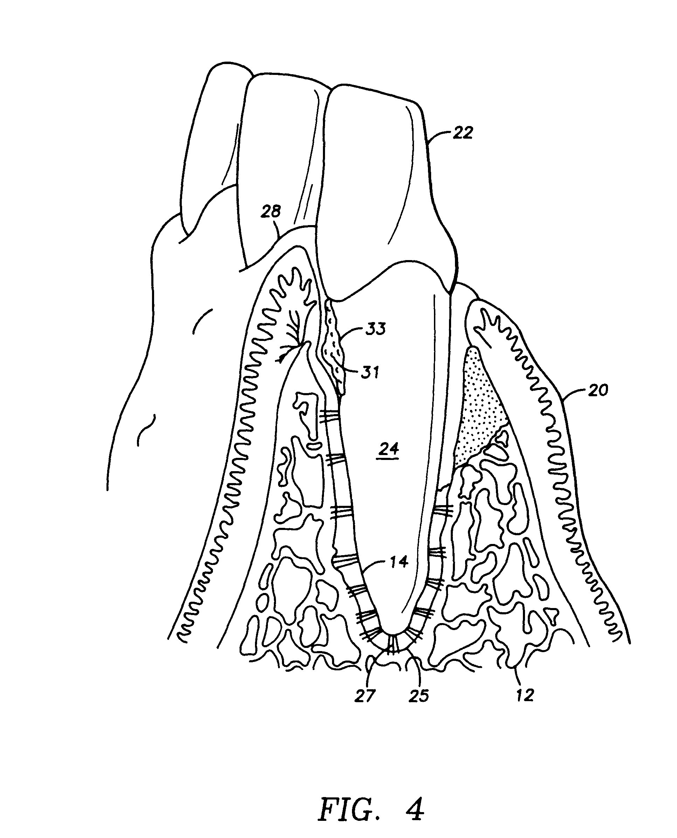

Referring to Figure 3, liposome 32 of the present invention is prepared from purified phospholipid, preferably phosphatidylcholine fraction, available from Sigma Chemical Co. of St. Louis, Missouri type IV-S, and a water soluble form of antibiotic, preferably Doxycycline. The selected antibiotic is first dissolved in .1 N sterile sodium chloride solution at a concentration of 1.0 to 2.5 grams of antibiotic per 100 milliliters of solution. Sterile 0.1 N saline solution is prepared by dissolving 5.58 grams of reagent grade sodium chloride in one liter of distilled water and filtering with a 0.45 micron disposable filtration unit. A disposable filtration unit, available from Nalge Co., P.O. Box 20365, Rochester NY 14602, may be used. Phosphatidycholine fraction is then added to the solution at a ratio of 1.0 to 10.0 per 100 milliliters of the NaCl-water solution. Antibiotic are incorporated as soluble salts at concentrations ranging from 0.25-2.5 grams per 100 milliliters based upon antibiotic potency. Routine formulations are prepared by dissolving 0.25 grams of antibiotic in ten milliliters of sterile saline solution in a sterile fifteen milliliter capped polystyrene centrifuge tube. Phosphatidylcholine, type IV-S, from soybean, and sold by Sigma Chemical Co. PO Box 14508, St Louis MO 63178, is weighed on a balance and added to the antibiotic solution at a concentration of 0.1 gram per milliliter. A Mettler Analytical balance available from Mettler Instrument Corporation, Box 71, Hightstown NJ 08520 may be used. The mixture in then shaken vigorously until an opalescent suspension is obtained and all waxy particulates of the phospholipid are gone. The shaking can be done manually or with a wrist-action mechanical shaker set at high speed, such as a wrist action shaker available from Burrell Corporation, 2223 5th Avenue, Pittsburgh PA 15219. The preparation of the opalescent suspension can be facilitated by heating the mixture to 55° C if necessary. Further liposome encapsulation is obtained by sequentially freezing the mixture in liquid nitrogen followed by rapid thawing in warm water for 2-4 cycles. If liquid nitrogen is not available, a dry ice/methyl alcohol mixture can be used. The solution containing liposomes is then tested for sterility, then stored at 4°C in two to ten milliliters quantities. If the finished solution is held more than one week prior to use, it is frozen and thawed again two to four times before use. It is believed that the shelf life of the liposomes 32 in solution carrying the antibiotic is no more than six months.

The liposomes 32 which are formed from the mixing process have aqueous layers 38 interdisposed between lipid bylayers 40. The antibiotic is carried in the aqueous layers 38. As a result, as each lipid bylayer 40 breaks down, a small amount of antibiotic, carried in the newly exposed aqueous layer 38, is released from the liposome 32. Although liposomes 30 having an aqueous bilayer having been described, oil based or other bilayer media could be used without deviating from the scope of the invention.

Chimotherapeutic encapsulating liposomes 32 are mixed with hydroxyapatite powder and microfibrillar collagen. The collagen is pretreated with 2% glutaraldehyde for 2 hours then washed with sterile saline solution three to four times to form a restorative bone paste 90. Hydroxyapatite in powder form, having a size of preferably 40 to 60 mesh, is mixed with equal amounts of the antibiotic encapsulated lipisomes 32 and the pretreated collagen. Zero point five (0.5) grams of hydroxyapatite is mixed with .05 to .08 ml. of liposome for a small pocket. For a medium pocket 30, 1.0 grams of hydroxyapatite is used, and for a large pocket 30, 1.5 grams of hydroxyapatite is used, with the same amount of liposome 32. More or less liposome may be used to form a paste. 100 mg. of collagen is added to the liposome hydroxyapatite mixture, and stirred to form a doughy paste. The paste is relatively thick, and easily formed on the resorbed area of the bone.

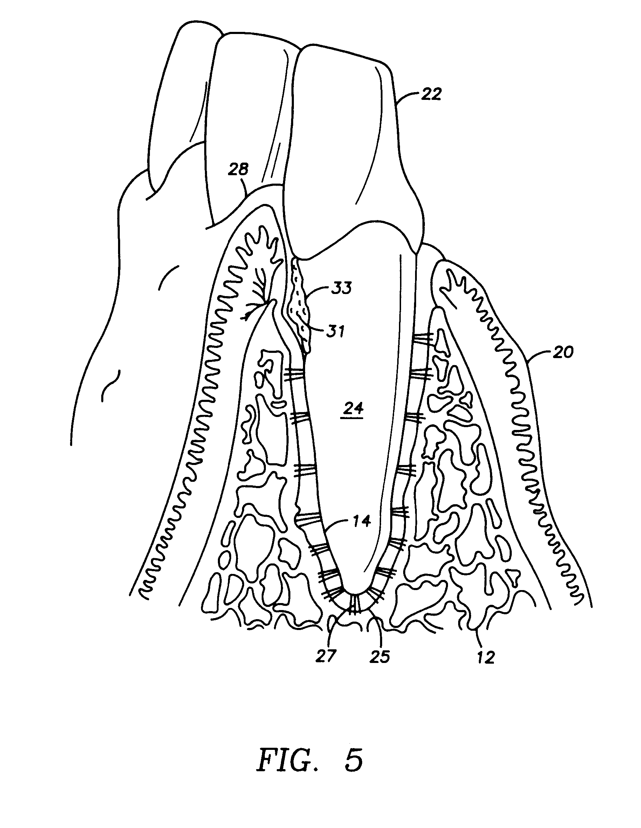

Referring to Figures 4, 5 and 6, to treat the mouth to restore the resorped bone, gum 20 is cut adjacent pocket 30 using a modified Widman flap 94. The Widman flap is formed by cutting through the gum 20 at the next adjacent tooth 15 from the infected tooth 14 and cutting down to the area of the gum 20 adjacent the base of the tooth root 24. A triangular flap 94 is then peeled back to expose the infection site 98. The epithelial lining and diseased tissues adjacent the pocket 30 are removed, and paste 90 is placed into the resorbed area 100 in Jawbone 12 and sculpted to form the profile of a non-resorbed bone. The flap 94 is then sutured shut. It has been found that in a periodontal pocket having resorbed bone and an 8mm pocket depth, four weeks after initial treatment with the liposome/collagen/ hydroxyapatite paste 90, the pocket was restored to a normal 3mm crevicular depth without reinfection. Use of antibiotic carrying liposomes on active periodontal sites has yielded a substantial increase in the duration of the presence of the antibiotic at the infection site. By extending the presence of the antibiotic on site, the duration of time before the infection returns will be extended, giving the patient and the clinician a longer period of time in which the body can reconstruct the resorbed area with paste 90 to help eliminate the pocket 30.

The presence and effect of the chemotherapeutic agent carrying liposomes 32 vis-a-vis prior art techniques was evaluated by comparing the presence of Doxycyctine, the antibiotic used for evaluation, the microscopic evaluation, or dark field, of the concentration of bacterial flora in untreated control sites, sites treated by prior art techniques, and sites treated by the liposome method. In the dark field evaluation, the concentration of the flora and the presence or absence of motile bacteria forms, primarily spirochetes, is evaluated. A dark field of +1 indicates the presence of a low level bacterial activity. A dark field of +2 indicates increased active bacterial activity, typically a coccal form bacteria, but no motile forms and no spirochetes. A dark field of +3 indicates the presence of coccal forms, motile forms, vacillary forms and spirochetes required for synergism and an active bacterial infection. A dark field of +4 indicates a fulminating +3 condition.

For evaluation purposes, the teeth of two separate individuals were evaluated. In the first individual, three different teeth were evaluated. The first tooth had 5mm pocket 30 depth, and acted as an untreated control. This untreated control site maintained a relatively constant anaerobic flora concentration and a relatively constant dark field evaluation during the four days it was evaluated. The second tooth of the first individual had a pocket depth of 5mm, with a +2. Upon treatment with Doxycycline only, the original darkfield of +2 was reduced to +1 after the first day, and returned to +2 after two days. Doxycycline could not be detected at the site one day after treatment. The third tooth of the first individual had an 11mm deep active pocket 30 with a +3 evaluation. It was treated with liposomes 32 carrying Doxycycline. The original darkfield of +3 was reduced to a +2 after two days, and the Doxycycline was detected on the first day after treatment, but could not be detected on the second day after treatment.

Two teeth were treated in the second individual. A control tooth, having a 7mm pocket depth and a +3 infection was reviewed for six days. The darkfield remained a constant +3 throughout the six day period. A second tooth was treated with a single dosage of Doxycycline and followed for five days. The concentration of anaerobic flora dropped significantly and the dark field improved from +3 to +1 during the first 24 hours after treatment. Concentration of cultivatable organisms were reduced by one logarithmic unit. This reduced level was maintained for 2 days without further treatment, but returned to pretreatment levels thereafter. After two day, the Doxycycline could not be detected on site, and motile forms returned. The darkfield climbed to a +2 on the third day after treatment, and returned to the original +3 condition on the fifth day.

In the same pocket 30 of the second individual treated with liposomes carrying Doxycycline, an original darkfield of +4 was reduced to a +2 value by the second day after treatment, and was further towered to a +1 value on the third and fourth days. The flora concentration was reduced by one logarithmic unit for two days after treatment, which returned to pretreatment levels after three days. However, motile species required for synergism remained absent, and liposomes and Doxycycline were detected in the pocket for four days after treatment and no further evaluation was performed.

Liposomes carrying antiviral or antifungal agents, or antibiotics may also be used to treat topical or peripheral infection sites, such as localized staph infections, or outbreaks such as those occurring from Herpes simplex. Further, such liposomic carriers could be used topically in combination wits systemic treatment, to control extreme staph infections.

Several different antibiotics have been, or may be, used with the liposomes of the present invention. These include:

| Antibiotic | Source |
|---|---|
| **Ciprofloxacin Hydrochloride** | |
| 1-Cyclopropyl-6-Fluoro-1,4-Dyhydro-4-oxo-7-(1-Piperanzyl)-3-Quinoline Carboxylic Acid | MILES INC. PHARMACEUTICAL DIVISION 400 Morgan Lane West Haven CT 06516 |
| **Doxycycline** (Vibramycin R) | |
| X-6-Deoxy-5-Oxytetracycline | PFIZER, INC. 235 E. 42nd Street New York, NY 10017 |
| **Gentamicin Sulfate** (Garamycin R) Injectable (Aminoglycoside) | S C H E R I N G PHARMACEUTICAL CORPORATION Galloping Hill Rd Kenilworth NJ 07033 |
| **Ampicillin**, Sodium/Sulbactam, Sodium (Unasyn R) | WARNER CHILCOT LABS 210 Tabor Rd Morris Plaines NJ 07950 |
| Monosodium (2S, 5R, 6R)-6-[(R)-2-Amino-2- Phenylacetamido] m-Dimethyl-7-oxo-4-Thia-1-Azabicyclo[3.2.0] Heptane-2-Carboxylate | ROERIG 235 E. 42nd St. New York NY 10017 |
| **Sulbactam**, sodium (2S, 5R)-3, 3-Dimethyl-7-oxo-4-Thia-1-Azabicyclo [3.2.0] Heptane-2-Carboxylate 4,4-Dioxide | |

Hydroxyapatite, also sold as hydroxyapatite, may be purchased from CeraMed Corporation, 12860 W. Ceder Drive, Lakewood CO 80228, under the trade name OSTEOGRAPH/P Periodontal Hydroxyapatite. The hydroxyapitite is a calcium phosphate material, typically having the chemical structure $Ca_{10}(PO_4)_6(OH)_2$.

The use of liposomes as a chemotherapeutic delivery vehicle lessens the need for systemic antibiotic treatment for periodontal disease and permits long duration treatment of periodontal infection sites with minimal loss of antibiotic out of the infection site while reducing the need for surgical intervention. When combined, in mixture, with collagen and hydroxyapatite, the resulting paste creates a non-infectious environment for bone regrowth on the resorbed surface and a stable matrix for supporting the hydroxyapatite during regeneration. Use of the liposome/hydroxyapatite/collagen mixture results in restoration of the bone, and reattachment of the gum at the proper normal point on the gum to eliminate the periodontal pocket. It is also believed that the liposomes may be combined with hydroxyapatite only to regrow bone in the periodontal pocket. The liposome 12 will help bind together the individual particles of hydroxyapatite, permitting the body to acclimate them and begin growing new bone in the defect. The gradual release of the antibiotic will help prevent reinfection during the acclimation period. Likewise, where infection is not carrying antibiotic, it may be possible to employ hydroxyapatite and liposomes without a chemotherapeutic agent.

It should be appreciated that the liposomes having chemotherapeutic agents may be used on any topical or peripheral infection site without deviating from the scope of the invention. The advantages of the present invention, high localized dosage to limited sites, is equally available to topical infection sites in areas other than the oral cavity, such as the skin.

## Claims

1. A composition for restoring a jawbone defect resulting from bone resorption in the jawbone due to periodontal disease, comprises a paste formed of an admixture of liposomes and hydroxyapatite.

2. A composition as claimed in claim 1 wherein the liposomes comprise aqueous layers disposed between lipid bilayers.

3. A composition as claimed in claim 1 wherein the liposomes have carrier layers disposed between lipid bilayers.

4. A composition as claimed in claim 3 wherein the liposomes are multilamellar liposomes.

5. A composition as claimed in claim 3 or 4 wherein the carrier layers are aqueous.

6. A composition as claimed in claim 1 or 2 wherein a chemotherapeutic agent is incorporated in the liposomes.

7. A composition as claimed in any one of claims 3 to 5 wherein a chemotherapeutic agent is incorporated in the carrier layer.

8. A composition as claimed in any one of claims 1 to 6 wherein the hydroxyapatite has a size of 40 to 60 mesh.

9. A composition as claimed in any one of claims 1 to 8 further comprising microfibrillar collagen.

10. A treatment and delivery system for the topical treatment of periodontal disease, comprising;
    a multilamellar body having carrier layers interdisposed between lipid bilayers; and,
    a chemotherapeutic agent disposed in said carrier layers;
    said lipid bilayers being sheddable over time to release said carrier layers over a period of time.

11. The system of claim 10, wherein said multilamellar body is a liposome.

12. The system of claim 10, wherein said chemotherapeutic agent is an antibiotic.

13. The system of claim 10, wherein said carrier layers are aqueous.

14. The system of claim 10, wherein said carrier layers are oil based.

*FIG. 1*

*FIG. 3*

*FIG. 6*

FIG. 2

FIG. 4

FIG. 5

**European Patent Office**

# EUROPEAN SEARCH REPORT

Application Number

| DOCUMENTS CONSIDERED TO BE RELEVANT | EP 91309055.1 |
|---|---|

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 107, no. 25, December 21, 1987 Columbus, Ohio, USA B.R. HEYWOOD et al. "An ultra-structural study of calcium phosphate formation in multi-lamellar liposome suspension" pages 521-522, abstract-no. 233 652g & Calcif. Tissue Int. 1987, 41(4), 192-201 | 1-5 | A 61 K 6/00 A 61 K 9/127 |
| A | WO - A1 - 90/01 923 (TECHNOLOGY UNLIMITED INC.) * Claims 1,4 * | 1-3, 10,11, 13 | |
| A | US - A - 4 906 670 (HIGASHI et al.) * Claims 1,3,5 * | 1,9, 11 | |
| A | EP - A2 - 0 302 847 (THE UNIVERSITY OF MARYLAND AT BALTIMORE) * Claims 1,8 * | 1,8,9 | TECHNICAL FIELDS SEARCHED (Int. Cl.5) A 61 K 6/00 A 61 K 7/00 A 61 K 9/00 A 61 L 25/00 |

The present search report has been drawn up for all claims

| Place of search | Date of completion of the search | Examiner |
|---|---|---|
| VIENNA | 14-11-1991 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document